# EUROPEAN PATENT APPLICATION

(11) **EP 3 945 095 A1**
(43) Date of publication of application: **02.02.2022**
(21) Application number: 20188209.9
(22) Date of filing: 28.07.2020
(51) Int. Cl.: C07K 14/745, B01D 15/08, A61K 38/48, C12N 9/64

(54) **A PROCESS FOR THE PURIFICATION OF PROTHROMBIN COMPLEX CONCENTRATE (PCC) AND FIX FROM COMPLETE PLASMA OR CRYO-POOR PLASMA**

(71) Applicant: BIA SEPARATIONS d.o.o., 5270 Ajdovscina (SI)
(72) Inventor: Josic, Djuro, 5270 Ajdoscina (SI); Begic, Marija, 5270 Ajdoscina (SI); Andjelkovic, Uros, 5270 Ajdoscina (SI)
(74) Representative: dompatent von Kreisler Selting Werner - Partnerschaft von Patent- und Rechtsanwälten mbB

(57) **Abstract**

A process for the purification of Prothrombin Complex Concentrate (PCC) from complete plasma or cryo-poor plasma using chromatography, the method comprising performing an initial sample displacement chromatography step of complete plasma or cryo-poor plasma, in particular undiluted cryo-poor plasma, on an anion exchanger to obtain a first fraction enriched in PCC.

A fraction comprising PCC and factor IX obtainable by the process of the invention.

## Description

The invention pertains to a process for the purification of Prothrombin Complex Concentrate (PCC) and FIX from complete plasma or cryo-poor plasma using chromatography, a fraction comprising PCC and a fraction comprising FIX obtainable by the process of the invention.

Prothrombin complex concentrate (PCC), also known as factor IX complex, is a medication made up of blood clotting factors II, IX, and X.[1] Some versions also contain factor VII.[2] It is used to treat and prevent bleeding in hemophilia B if pure factor IX is not available.[1][3] It may also be used in those with not enough protein K dependent clotting factors and inhibitors due to other reasons such as warfarin therapy.[3] It is given by slow injection into a vein by a slow intravenous injection".[1] Prothrombin complex concentrate came into medical use in the 1960s.[5] It is on the World Health Organization's List of Essential Medicines, the safest and most effective medicines needed in a health system.[6][7] It is made from human plasma.[4] A version that is made by recombinant methods which only contains factor IX is also available.[8]

PCC reverses the effects of warfarin and other vitamin K antagonist anti-coagulants and is used in cases of significant bleeding in people with a coagulopathy. It is also used when such a person must undergo an emergency operation treatment [9]. Other uses include a deficiency of one of the included clotting factors, either congenital or due to liver disease, and haemophilia B.[9] Several guidelines, including those from the American College of Chest Physicians, recommend PCC for warfarin reversal in people with serious bleeding.[10][11][12][13].

EP 0549964 A2 discloses a method for producing a virus-inactivated prothrombin complex concentrate (PCC preparation) with vitamin K dependent clotting factors prothrombin (factor II), proconvertin (factor VII), Stuart-Prower factor (factor X) and hemophilia B factor (factor IX), as well as vitamin K dependent clotting inhibitors protein C, protein S and protein Z wherein stabilized cryopoor plasma is chromatographed on ion exchange materials. After cryoprecipitation and removal of cryoglobulins,citrate-stabilized plasma is treated with an anion exchange material based on QAE-modified dextran. After elution, the resulting fraction is subjected to a first ultrafiltration with a suitable buffer, the so obtained crude PPSB extract is chromatographed on DEAE modified dextran or agarose (Sepharose), and then eluted with a suitable buffer.

### Summary of The Invention

The object underlying the present invention is solved by a process utilizing principles of sample displacement chromatography. Such chromatography is employed with a monolithic anion exchanger separation media at an initial stage of the purification process.

Subject matter of the invention is a process for the purification of Prothrombin Complex Concentrate (PCC) from complete plasma or cryo-poor plasma, in particular undiluted cryo-poor plasma, by means of chromatography using a monolithic stationary phase, the method comprising
a) performing of an initial sample displacement chromatography step of complete plasma or cryo-poor plasma on an anion exchanger to obtain a first fraction enriched in PCC.
   In an embodiment of the present invention the initial chromatography step may be performed with a weak anion exchanger or a strong anion exchanger. In another embodiment of the invention the process can further comprise
b) after elution of the first fraction enriched in PCC from the initial sample displacement chromatography, a second chromatography step of the first fraction enriched in PCC, wherein the chromatography step is performed with an anion exchanger to obtain a second fraction enriched in PCC.
   In yet another embodiment of the invention the process may further comprise
c) a third chromatography step of the second fraction enriched in PCC, wherein the chromatography step can be performed with a cation exchanger to obtain a third fraction comprising enriched FIX, and in which fraction other components of the PCC, i. e. FII, FVII, and FX, can be depleted or removed.

Due to the unexpected finding that undiluted cryopoor plasma can be employed by performing the method of the invention the present method is advantageous over prior art as referenced in [15] which demand dilution of plasma at least two times, most frequently even five times. This saves loss of activity of physiologically active proteins.

In still another embodiment of the invention the cation exchanger may be provided with -SO₃, -SO₄, or -PO₄ groups. Elution from the SO₄ (sulphate) monolith can be performed either by salt or by pH gradient.

In a further embodiment of the invention the anion and/or cation exchanger of the second and/or third chromatography step can be bulk materials or monolithic materials.

An embodiment of the invention may comprise a solvent/detergent virus inactivation step. The solvent/detergent virus inactivation step can be performed prior to the third chromatography step.

Another embodiment of the invention can provide an addition of a stabiliser to at least one of the first, second and third fractions, the stabiliser can be in particular a positively charged amino acid such as lysine. At this stage of the process of the invention heparin may be added to at least one of the first, second and third fraction.

A still further embodiment of the invention can provide a virus filtration step which may be performed with at least one of the first, second and/or third fraction.

Subject matter of the invention is also a fraction comprising FIX obtainable by the process comprising the following steps:
(1) providing an undiluted cryo-poor plasma;
(2) performing a sample displacement chromatography with the undiluted cryo-poor plasma on a monolithic weak or a strong anion exchanger material such as DEAE or Q monolithic material with about 6 µm pores;
(3a) washing the weak or strong anion exchanger monolithic material with a first NaCl buffer having about 100 mM to about 300 mM, in particular about 200 mM NaCl;
(3b) followed by washing with a higher second NaCl buffer having about 200 mM to about 350 mM in particular about 280 mM NaCl;
(3c) elution with a NaCl buffer having about 1 M to about 2.5 M NaCl, in particular about 2 M NaCl; thereafter
(4) performing a chromatography on a weak or strong anion exchange material; optionally using parts of the fraction obtained after step (4) for PCC manufacturing;
(5) stabilization with about 0.1% L-lysine monochloride;
(6) virus inactivation with about 0.3% (w/w) TnBP, about 1% (w/w) Tween80, 6-8 h at 26-28oC;
(7) cation-exchange chromatography;
(8) virus filtration on CIM monolith;
(9) addition of about 0.1 IU heparin per 1IU FIX,
(10) optionally isolating an eluate from the cation exchange material.

The fraction of the invention comprising factor IX may be characterised by the following features: factor IX specific activity higher than 120 IU/mg protein. Other protein K dependent clotting factors and inhibitors are, if traces present, below their detection limit.

Another subject matter of the invention is a fraction comprising PCC obtainable by a process comprising the following steps:
(1) providing an undiluted cryo-poor plasma;
(2) performing a sample displacement chromatography with the undiluted cryo-poor plasma on a monolithic weak or a strong anion exchanger material such as DEAE or Q monolithic material with about 6 µm pores;
(3a) washing the weak or strong anion exchanger monolithic material with a first NaCl buffer having about 100 mM to about 300 mM, in particular about 200 mM NaCl;
(3b) followed by washing with a higher second NaCl buffer having about 200 mM to about 350 mM in particular about 280 mM NaCl;
(3c) elution with a NaCl buffer having about 600 mM to about 2.5 M NaCl, in particular about 1 2 M NaCl; thereafter
(4) performing a chromatography on a weak or strong anion exchange material such as CIM DEAE chromatography material,
(5) optionally isolating an eluate from the weak or strong anion exchange material.

The fraction of the invention comprising PCC can be characterised by the following features, clotting factor IX specific activity between 2 and 10 IU/mg protein and correspondingly balanced concentration of both vitamin K dependent clotting factors and inhibitors.

### Detailed Description of The Invention

In ion-exchange chromatography, the interaction of the solute ions and the stationary phase based on their charges determines which ions will bind and to what degree. When the stationary phase features positive groups which attracts anions, it is called an anion exchanger; when there are negative groups on the stationary phase, cations are attracted, and it is a cation exchanger. The attraction between ions and stationary phase also depends on the resin, organic particles used as ion exchangers.

Anion-exchange chromatography is a process that separates substances based on their charges using an ion-exchange resin containing positively charged groups, such as diethyl-aminoethyl groups (DEAE). In solution, the resin is coated with positively charged counter-ions (cations). Anion exchange resins will bind to negatively charged molecules, displacing the counter-ion. Anion exchange chromatography is commonly used to purify proteins, amino acids, sugars/carbohydrates and other acidic substances with a negative charge at higher pH levels. The tightness of the binding between the substance and the resin is based on the strength of the negative charge of the substance.

The artisan discriminates two different kinds of anion exchangers, strong and weak anion exchanger. A "strong" ion exchanger will not lose the charge on its matrix once the column is equilibrated and so a wide range of pH buffers can be used. "Weak" ion exchangers have a range of pH values in which they will maintain their charge. If the pH of the buffer used for a weak ion exchange column goes out of the capacity range of the matrix, the column will lose its charge distribution and the molecule of interest may be lost. Despite the smaller pH range of weak ion exchangers, they are often used over strong ion exchangers due to their having greater specificity. In some experiments, the retention times of weak ion exchangers are just long enough to obtain desired data at a high specificity.

Resins (often termed 'beads') of anion exchange columns may include functional groups such as weak/strong bases. Some examples of functional groups of strong ion exchange resins are quaternary ammonium cation (Q), which is an anion exchanger, and sulfonic acid (S, -SO₂OH), which is a cation exchanger. These types of exchangers can maintain their charge density over a pH range of 0-14. Examples of functional groups of weak ion exchange resins include diethylaminoethyl (DEAE, -C₂H₄N(CH₂H₅)₂), which is an anion exchanger, and carboxymethyl (CM, -CH₂-COOH), which is a cation exchanger. These two types of exchangers can maintain the charge density of their columns over a pH range of 5-9.

According to the invention the Prothrombin Complex Concentrate (PCC) from complete plasma or cryo-poor plasma is purified by using chromatography by performing an initial sample displacement chromatography step of complete plasma or cryo-poor plasma, in particular undiluted cryo-poor plasma, on a monolithic anion exchanger to obtain a first fraction enriched in PCC.

According to the invention, the initial chromatography step is performed with a monolithic weak anion exchanger or strong anion exchanger.

US 5,453,185 A discloses monolithic separation materials. Monoliths can be regarded as alternative to particle-based stationary phases in chromatography. Both silica and polymer monoliths are able to perform fast separations without significant loss in separation efficiency. Particularly useful are DEAE or Q ion-exchange monoliths with 6µm pores.

According to the invention it is critical to adjust loading conditions to enable sample displacement chromatography. Sample displacement chromatography (SDC) in reversed-phase and ion-exchange modes was introduced approximately twenty years ago. This method takes advantage of relative binding affinities of components in a sample mixture. At the beginning of the chromatographic separation, a mixture of solutes to be separated - complete plasma or cryo-poor plasma in case of the invention - is applied to the column with monolithic separation material, under conditions selected to promote high retention. The higher-affinity solutes are preferentially retained near the head of the column, with the lower-affinity solutes moving farther downstream. The fastest moving component begins to form a pure zone downstream. The other components also begin to form zones, but the continued supply of the mixed feed at head of the column prevents full resolution. During loading, there is a competition among different sample components for the sorption on the surface of the stationary phase.

SDC was first used for the preparative purification of proteins. Later, it was demonstrated that this kind of chromatography can also be performed in ion-exchange, affinity and hydrophobic-interaction mode. It has also been shown that SDC can be performed on monoliths and membrane-based supports in both analytical and preparative scale. Recently, SDC in ion-exchange and hydrophobic interaction mode was also employed successfully for the removal of trace proteins from monoclonal antibody preparations and for the enrichment of low abundance proteins from human plasma. In this review, the principals of SDC are introduced, and the potential for separation of proteins and peptides in micro-analytical, analytical and preparative scale is discussed.[14]

After loading a wash of the weak or strong anion exchanger, in particular the monolithic material, can be performed with a first NaCl buffer having about 100 mM to about 300 mM, in particular about 200 mM NaCl; followed by washing with a higher second NaCl buffer having about 200 mM to about 350 mM in particular about 280 mM NaCl.

An elution of a first fraction is performed with a NaCl buffer having about 1 M to about 2.5 M NaCl, in particular about 2 M NaCl.

After elution of the first fraction enriched in PCC from the initial sample displacement chromatography, a second chromatography step of the first fraction enriched in PCC, is performed with an anion exchanger using bulk or monolithic separation material, in particular monolitic CIM DEAE or Q material, to obtain after elution a second fraction enriched in PCC. The eluate can be used for PCC production.

The second fraction enriched in PCC can be subjected to a third chromatography step to obtain FIX. The third chromatography step is performed with a cation exchanger to obtain a third fraction comprising enriched FIX, and other components of the PCC, i. e. FII, FVII, and FX, are depleted or removed.

The cation exchanger may be provided with -SO₃, -SO₄, or -PO₄ groups. As the anion exchanger also the cation exchanger can be particulate material or monolithic material.

A solvent/detergent virus inactivation step is mandatory for therapeutical use due to regulatory requirements. The solvent/detergent virus inactivation step can be performed prior to the third chromatography step. A suitable method for virus inactivation is described in EP 0 131 740 A2.

Addition of a stabiliser to at least one of the first, second and third fractions is advantageous. The stabiliser is in particular a positively charged amino acid such as lysine. At this stage of the process of the invention also heparin may be added to at least one of the first, second and third fraction.

If required by regulatory orders a virus filtration [16] step may be performed with at the least one of the first, second and/or third fraction.

The method of the invention yields a fraction comprising FIX. The respective FIX containing fraction is obtainable by the process comprising the following steps:
(1) providing an undiluted cryo-poor plasma;
(2) performing a sample displacement chromatography with the undiluted cryo-poor plasma on a monolithic weak or a strong anion exchanger material such as DEAE or Q monolithic material with about 6 µm pores;
(3a) washing the weak or strong anion exchanger monolithic material with a first NaCl buffer having about 100 mM to about 300 mM, in particular about 200 mM NaCl;
(3b) followed by washing with a higher second NaCl buffer having about 200 mM to about 350 mM in particular about 280 mM NaCl;
(3c) elution with a NaCl buffer having about 1 M to about 2.5 M NaCl, in particular about 2 M NaCl; thereafter
(4) performing a chromatography on a weak or strong anion exchange material; optionally using parts of the fraction obtained after step (4) for PCC manufacturing;
(5) stabilization with about 0.1% L-lysine monochloride;
(6) virus inactivation with about 0.3% (w/w) TnBP, about 1% (w/w) Tween80, 6-8 h at 26-28oC;
(7) cation-exchange chromatography;
(8) virus filtration on CIM monolith;
(9) addition of about 0.1 IU heparin per 1IU FIX,
(10) optionally isolating an eluate from the cation exchange material.

The fraction of the invention comprising factor IX may be characterised by the following features: factor IX specific activity higher than 120 IU/mg protein, and other protein K dependent clotting factors and inhibitors are, if present, below their detection limit [16].

The method of the invention yields a fraction comprising PCC. The respective PCC containing fraction is obtainable by a process comprising the following steps:
(1) providing an undiluted cryo-poor plasma;
(2) performing a sample displacement chromatography with the undiluted cryo-poor plasma on a monolithic weak or a strong anion exchanger material such as DEAE or Q monolithic material with about 6 µm pores;
(3a) washing the weak or strong anion exchanger monolithic material with a first NaCl buffer having about 100 mM to about 300 mM, in particular about 200 mM NaCl;
(3b) followed by washing with a higher second NaCl buffer having about 200 mM to about 350 mM in particular about 280 mM NaCl;
(3c) elution with a NaCl buffer having about 600 mM to about 2.5 M NaCl, in particular about 1 2 M NaCl; thereafter
(4) performing a chromatography on a weak or strong anion exchange material such as CIM DEAE chromatography material,
(5) optionally isolating an eluate from the weak or strong anion exchange material.

The fraction of the invention comprising PCC can be characterised by the following features: clotting factor IX specific activity between 2 and 10 IU/mg protein and correspondingly balanced concentration of both vitamin K dependent clotting factors and inhibitors.

The invention is further illustrated by the following non-limiting examples.

### Example 1

### Analytical Methods:

Assay for determination of FIX activity performed as published in [16] as well as immunoblot with monoclonal and/or polyclonal antibodies as well as protein identification by LC-MS/MS - clotting factor IX. FIX and other proteins see [17].

### Example 2

Cryo poor plasma was obtained from Institute of Transfusiology, Zagreb, Croatia. Donations were tested for virus safety (Enveloped and non-enveloped blood borne viruses) after agreement of non-commercial donors and approval of the Institute's Ethical Committee. After centrifugation and filtration, plasma was tempered at 22°C and applied directly to the monolithic chromatographic column.
Sample: Fresh frozen undiluted plasma, conductivity; σ∼ 12 mS/cm

### Sample preparation:

1) Plasma centrifugation at 10000 rpm, 20 min at 4°C.
2) Filtration on 5µm membrane (Milipore, MA, USA).

### Example 3

### Manufacturing of PCC

265 mL of undiluted cryo-poor plasma was subjected to a sample displacement chromatography on a monolithic weak or a strong anion exchanger material such as DEAE or Q monolithic material (DEAE CIM column, 8 mL) with about 6 µm pores, (BIA Separations d.o.o., Ajdovscina, Slovenia). After application of the cryo poor plasma the anion exchanger monolithic material was first washed with a 200 mM NaCl buffer five column volumes, followed by washing with a higher second buffer having 280 mM NaCl five column volumes The PCC was eluted with 2 M NaCl. The collected PCC was subjected to chromatography on CIM DEAE and the PCC eluted. The obtained PCC showed a clotting factor IX specific activity of five - 5 IU/mg protein and correspondingly balanced concentration of both vitamin K dependent clotting factors and inhibitors.

### Example 4

### Manufacturing of FIX

265 ml undiluted cryo-poor plasma was subjected to a sample displacement chromatography on DEAE monolithic material (DEAE CIM column, 8 mL) with about 6 µm pores, (BIA Separations d.o.o., Ajdovscina, Slovenia). After application of the cryo poor plasma the anion exchanger monolithic material was first washed with a 200 mM NaCl buffer [five (5 CV) column volumes], followed by washing with a higher second buffer having 280 mM NaCl [five (5CV) column volumes]. The PCC fraction eluting with 2 M NaCl buffer was subjected to a chromatography on DEAE monolithic material. The obtained fraction was stabilized with 0.1% L-lysine. Subsequently, a virus inactivation according to by a solvent/detergent method was performed [18] for example with 1% (v/w) Tween 80 and 0,3% (v/w) TnBP.

### Cation-Exchange Chromatography:

Sample: Human prothrombin complex after two DEAE chromatographic steps on CIM column (see Example 3). The eluate containing 20 IU of clotting factor IX (4 mg protein) was loaded on CIMmic sulphate SO4 0.2ml monolithic column, Äkta Explorer chromatographic system, 2 ml sample loop.

BCA Protein Assay kit, Thermo Scientific Pierce^{™}, Cat. #23225/23227. Monoclonal Anti-Factor IX antibody produced in mouse, Sigma-Aldrich, Darmstadt, Germany, Cat. # F2645.

Goat Anti-Mouse IgG, HRP conjugate, Milipore, MA, USA, Cat. # 12-349.

Factor X Polyclonal Antibody produced in rabbit, ThermoFisher, IL, USA, Cat. # PA5-29118.

Goat Anti-Rabbit IgG, HRP conjugate, Milipore, MA, USA, Cat. # 12-348. Anti-Factor VII antibody, Mouse monoclonal, Sigma-Aldrich, #F8146. Prothrombin Monoclonal Antibody, Invitrogen, #LF-MA0171.

### Detection methods:

Assay for determination of FIX activity see [16].

Immunoblot with monoclonal and/or polyclonal antibodies as well as protein identification by LC-MS/MS - clotting factor IX - Reference above (Haemophilia), FIX and other proteins, see reference [19].

### Chromatography:

Buffers and elution sequence:
Equilibration: Buffer A, 400mM NaCl, 20mM phosphate buffer, pH 5, 39 mS/cm, Wash: Buffer A, 10 column volumes
Elution: Fraction 1, Buffer 1: 300 mM NaCl, 20mM phosphate buffer pH 5, 30.4 mS/cm, 5 column volumes,
Fraction 2, Buffer 2: 250 mM NaCl, 20mM phosphate buffer , pH 5.03, 26 mS/cm, 5 column volumes,
Fraction 3, Buffer 3: 250 mM NaCl, 20mM phosphate buffer, pH 6, 25.9 mS/cm, 5 column volumes,
Fraction 4, Buffer 4: 250 mM NaCl, 20mM phosphate buffer, pH 7.2, 25.9 mS/cm 5 column volumes,
Fraction 5, Buffer 5: 50 mM NaCl, 20mM phosphate buffer, pH 7.225, 7.76 mS/cm 5 column volumes,
Fraction 6, Buffer 6: 20 mM NaCl, 20mM phosphate buffer, pH 7.2, 4.7 mS/cm, 5 column volumes,
Fraction 7, Buffer 7: 10 mM phosphate buffer, pH 8, 1.63 mS/cm, 10 column volumes.

It has been found that column equilibration with 400mM NaCl, 20mM phosphate buffer, pH 5, show the best results when FIX purification comes to question.

Majority of FIX is eluted in fraction 4, with 250 mM NaCl, 20mM phosphate buffer, pH 7.2. Immunoblots indicate FX presence in that same fraction 4 in a very small amount, but no FX clotting activity could be detected in this fraction. FIX yield was 70-80%. The specific activity of clotting factor IX was about 190 IU/mg protein. Theoretic specific activity is about 250IU/mg protein [16].

Factors II and VII do not bind to the column in given conditions. Other impurities were not analysed. The main impurity is usually vitronectin [16].

The virus filtration on CIM monolith is described in [16].

The factor IX obtained has a specific activity higher than 150 IU/mg protein. Other protein K dependent clotting factors and inhibitors are, if present, below their detection limit.

"All references cited herein are incorporated by reference to the full extent to which the incorporation is not inconsistent with the express teachings herein."

### References

[1] World Health Organization (2009). Stuart MC, Kouimtzi M, Hill SR (eds.). WHO Model Formulary 2008. World Health Organization. pp. 259-60. hdl:10665/44053. ISBN 9789241547659.
[2] Perkins JC (2014). Hematology/Oncology Emergencies, An Issue of Emergency Medicine Clinics of North America. Elsevier Health Sciences. p. 720. ISBN 9780323320290.
[3] British national formulary: BNF 69 (69 ed.). British Medical Association. 2015. p. 171. ISBN 9780857111562.
[4] "Factor IX (Human), Factor IX Complex (Human)". The American Society of Health-System Pharmacists.
[5] Besa EC (1992). Hematology. Lippincott Williams & Wilkins. p. 276. ISBN 9780683062229.
[6] World Health Organization (2019). World Health Organization model list of essential medicines: 21st list 2019. Geneva: World Health Organization. hdl:10665/325771.
[7] The selection and use of essential medicines: Twentieth report of the WHO Expert Committee 2015 (including 19th WHO Model List of Essential Medicines and 5th WHO Model List of Essential Medicines for Children). WHO. 2015. p. 510. ISBN 9789240694941.
[8] "Factor IX (Recombinant)". The American Society of Health-System Pharmacists.
[9] Haberfeld, H, ed. (2015). Austria-Codex (in German). Vienna: Osterreichischer Apothekerverlag.
[10] "ACCP 2012 guidelines: 'Evidence-Based Management of Anticoagulant Therapy, Section 9.3 Treatment of Anticoagulant-Related Bleeding"'. Chest Journal.
[11] Haemostasis and Thrombosis Task Force for the British Committee for Standards in Haematology. Guidelines on oral anticoagulation: 3rd edition. Br J Haematol. 1998;101:374-387.
[12] Baker RI, Coughlin PB, Gallus AS, Harper PL, Salem HH, Wood EM (November 2004). "Warfarin reversal: consensus guidelines, on behalf of the Australasian Society of Thrombosis and Haemostasis". The Medical Journal of Australia. 181 (9): 492-7. PMID 1551619.
[13] Palareti G (1998). "A guide to oral anticoagulant therapy. Italian Federation of Anticoagulation Clinics". Haemostasis. 28 Suppl 1: 1-46. doi:10.1159/000054103. PMID 9820837.
[14] Sample displacement chromatography as a method for purification of proteins and peptides from complex mixtures; Martina Srajer Gajdosik,1 James Clifton,2 and Djuro Josic.
[15] Josic, D. Hoffer, L. et al. Manufacturing of a prothrombin complex concentrate aiming at low thrombogenicity, Thromb. Res. 100(5), (2000) 433-441.
[16] Josic D., Kannicht C., Löster K., Pock K., Iberer G., Buchacher A., "Vitronectin in clotting factor IX concentrates, Haemophilia 7 (3) (2001) 250-257, https://doi.org/10.1046/j.10365-2516.2001.00503.x.
[17] Clifton J., Huang F., Gašo-Sokač D., Brilliant K., Hixson D., Josic D.: Use of proteomics for validation of the isolation process of clotting factor IX from human plasma, Journal of Proteomics 73 (3) (2010) 678. doi:10.1016/j.jprot.2009.09.020.
[18] Josic D., Bal F., Schwinn, H.: Isolation of plasma proteins from clotting cascade by heparin affinity chromatography, Journal of Chromatography 632 (1993) 1-10. DOI:10.1016/0021-9673(93)80019-5.
[19] Clifton J., Huang F., Gašo-Sokač D., Brilliant K., Hixson D., Josic D.: Use of proteomics for validation of the isolation process of clotting factor IX from human plasma, Journal of Proteomics 73 (3) (2010) 678. doi:10.1016/j.jprot.2009.09.020

## Claims

1. A process for the purification of Prothrombin Complex Concentrate (PCC) from complete plasma or cryo-poor plasma, in particular undiluted cryo-poor plasma, by means of chromatography using a monolithic stationary phase, the method comprising
a) performing an initial sample displacement chromatography step of complete plasma or cryo-poor plasma on an anion exchanger to obtain a first fraction enriched in PCC.

2. The process of claim 1, wherein the initial chromatography step is performed with a weak anion exchanger or a strong anion exchanger.

3. The process of claim 1 or 2, wherein the method further comprises
b) after elution of the first fraction enriched in PCC from the initial sample displacement chromatography, a second chromatography step of the first fraction enriched in PCC, wherein the chromatography step is performed with an anion exchanger to obtain a second fraction enriched in PCC.

4. The process of claim 3, wherein the method further comprises
c) a third chromatography step of the second fraction enriched in PCC, wherein the chromatography step is performed with a cation exchanger to obtain a fraction comprising enriched FIX, and in which fraction other components of the PCC, i. e. FII, FVII, and FX, are depleted or removed.

5. The process of claim 4, wherein the cation exchanger has -SO₃, -SO₄, or -PO₄ groups.

6. The process of any one of the claims 1 to 5, wherein the anion and/or cation exchanger of the second and/or third chromatography step are bulk materials or monolithic materials.

7. The process of any one of the claims 1 or 5, further comprising a solvent/detergent virus inactivation step, preferably wherein the solvent/detergent virus inactivation step is performed prior to the third chromatography step.

8. The process of any one of the claims 1 to 7, wherein a stabiliser is added to at least one of the first, second and third fractions.

9. The process of claim 8, wherein the stabiliser is a positively charged amino acid such as lysine.

10. The process of any one of the claims 1 to 9, wherein a virus filtration step is performed with at least one of the first, second and third fraction.

11. The process of claim 8, wherein heparin is added to at least one of the first, second and third fraction.

12. A fraction comprising FIX obtainable by the process comprising the following steps:
(1) providing an undiluted cryo-poor plasma;
(2) performing a sample displacement chromatography with the undiluted cryo-poor plasma on a monolithic weak or strong anion exchanger material with about 6 µm pores;
(3a) washing the weak or strong anion exchanger monolithic material with a first NaCl buffer having about 100 mM to about 300 mM, in particular about 200 mM NaCl;
(3b) followed by washing with a higher second NaCl buffer having about 200 mM to about 350 mM, in particular about 280 mM NaCl;
(3c) elution with a NaCl buffer having about 1 M to about 2.5 M NaCl, in particular about 2 M NaCl; thereafter
(4) performing a chromatography on a weak or strong anion exchange material; optionally using parts of the fraction obtained after step (4) for PCC manufacturing;
(5) stabilization with about 0.1% L-lysine;
(6) virus inactivation
(7) cation-exchange chromatography;
(8) virus filtration on CIM monolith;
(9) addition of about 0.1 IU heparin per 1IU FIX,
(10) optionally isolating an eluate from the cation exchange material.

13. A fraction comprising PCC obtainable by a process comprising the following steps:
(1) providing an undiluted cryo-poor plasma;
(2) performing a sample displacement chromatography with the undiluted cryo-poor plasma on a monolithic weak or strong anion exchanger material such as DEAE or Q monolithic material with about 6 µm pores;
(3a) washing the weak or strong anion exchanger monolithic material with a first NaCl buffer having about 100 mM to about 300 mM, in particular about 200 mM NaCl;
(3b) followed by washing with a higher second NaCl buffer having about 200 mM to about 350 mM in particular about 280 mM NaCl;
(3c) elution with a NaCl buffer having about 600 mM to about 2.5 M NaCl, in particular about 1.2 M NaCl; thereafter
(4) performing a chromatography on a weak or strong anion exchange material such as CIM DEAE chromatography material,
(5) optionally isolating an eluate from the weak or strong anion exchange material.

14. The fraction of claim 12 comprising factor IX **characterised by** a specific activity higher than 120 IU/mg protein, preferably comprising PCC **characterised by** a clotting factor IX specific activity between 2 and 10 IU/mg protein and correspondingly balanced concentration of both vitamin K dependent clotting factors and inhibitors.
